# EUROPEAN PATENT APPLICATION

(11) **EP 2 551 354 A1**
(43) Date of publication of application: **30.01.2013**
(21) Application number: 11175266.3
(22) Date of filing: 25.07.2011
(51) Int. Cl.: C12Q 1/68

(54) **Functionalization of RNA oligonucleotides**

(71) Applicant: Universität Heidelberg, 69117 Heidelberg (DE)
(72) Inventor: Winz, Marie-Luise, 69123 Heidelberg (DE); Samanta, Ayan, 68167 Mannheim (DE); Benzinger, Dirk, 68163 Mannheim (DE); Jäschke, Andreas, 69118 Heidelberg (DE)
(74) Representative: Schüssler, Andrea

(57) **Abstract**

The inventors of the present invetion developed a two-step method for the post-synthetic enzymatic incorporation of chemical modifications or rather functionalization of RNA oligonucleotides for their labelling. In a first step, the RNA oligonucleotide is tailed by the incorporation of a convertible azido-modified (N₃-modified) nucleotide at the 3'-terminus in an enzymatic reaction with a nucleotidyl transferase, e.g., the enzyme poly(A) polymerase (PAP) and an N₃-modified nucleoside triphosphate (N₃-modified NTP). In a second step, the functionalization is accomplished with an azide-reactive group, e.g., via a copper-catalyzed 1,3-dipolar cycloaddition reaction between the incorporated N₃-modified nucleotide and a functional group containing a terminal alkyne moiety (copper-catalyzed click reaction), via a strain-promoted [3 + 2] dipolar cycloaddition between the incorporated N₃-modified nucleotide and a functional group containing a cyclooctyne moiety (strain-promoted click reaction), or via an azide-phosphine conjugation between the incorporated N₃-modified nucleotide and a functional group containing a phosphine moiety (Staudinger ligation). To achieve internal chemical modifications or rather functionalization of RNA oligonucleotides, an additional step can be introduced between the first and the second step, in which a second RNA oligonucleotide is enzymatically attached to the N₃-modified nucleotide at the 3'-terminus of the first step, thus converting the 3'-terminal N₃-modified nucleotide into an internal N₃-modified nucleotide that can be further converted in one of the aforementioned reactions.

## Description

### Technical field

The present invention concerns a method for the functionalization of RNA oligonucleotides, particularly for their labelling, by enzymatically incorporating a convertible azido-modified nucleotide at the 3'-terminus of the RNA oligonucleotide in a first step, which is in a second step conjugated with an azide-reactive group, e.g., with a terminal alkyne moiety by a copper-catalyzed click reaction, with a cyclooctyne moiety by a strain-promoted click reaction, or with a phosphine moiety by Staudinger ligation, wherein this 3'-terminal chemical modification can be converted into an internal chemical modification in an additional step between the first and the second step.

### Prior art

Ribonucleic acids (RNA) have been shown to be unexpectedly versatile in terms of function throughout the last few decades. Thus, in the post-genomic era, RNA oligonucleotides have become one of the most interesting objects of investigation in diverse fields, ranging from biophysics, biochemistry, molecular and cellular biology, to medicine and pharmacy, wherein RNA can serve as drug as well as drug target. In order to find new drug targets and new therapeutic approaches it is necessary to gain insight into the biological function of the diverse RNA oligonucleotide species e.g., their localization in living systems, their structure and dynamics. However, the research on the biological function of RNA oligonucleotide species often depends on labelling by functionalization, i.e. on the incorporation of functional groups into these studied RNA oligonucleotides. Therefore, it is often necessary to incorporate chemical modifications into the molecules.

One common class of functional groups are fluorescent dyes, which can be employed to study the folding of RNA by single molecule fluorescence resonance energy transfer (Zhuang, X. et al., Science, 2000, 288:2048-2051; Kobitski, A.Y. et al., Nucleic Acids Res., 2007, 35:2047-2059). Furthermore, biotin residues can be used to immobilize RNA, e.g. to perform atomic force spectroscopy (Vilfan, I.D. et al., Nucleic Acids Res., 2007, 35:6625-6639), whereas many other chemical modifications have been shown to modulate the properties of RNA species, such as delivery (Wolfrum C. et al., Nature Biotechnology, 2007, 25:1149-1157) or stability (Kubo, T. et al., Biochem. Biophys. Res. Commun. 2008, 365:54-61) of siRNAs.

The incorporation of chemical modifications or rather functionalization of RNA oligonucleotides for their labelling can be achieved during their synthesis or post-synthetically, wherein the synthesis of RNA oligonucleotides can be achieved via chemical synthesis, enzymatic synthesis or synthesis in the organism, where the synthesized RNA oligonucleotides are directly derived from the organism.

With regard to the incorporation of chemical modifications or rather functionalization of RNA oligonucleotides during the chemical synthesis of RNA oligonucleotides, the phosphoramidite method is used most widely at present (Koizumi, M. et al., Nucleic Acids Res., 1989, 17:7059-7071; Venkatesan, N. et al., Curr. Med. Chem., 2003, 10:1973-1991). In general, this phosphoramidite method makes use of a condensation reaction between a nucleoside phosphoramidite and a nucleoside as a key reaction using imidazole or tetrazole based chemical compounds as activators. However, the phosphoramidite method enables the direct incorporation of only a small subset of chemical modifications in RNA oligonucleotides during their synthesis because of the instability of many functional groups towards standard phosphoramidite coupling conditions. For the synthesis of long site-specifically modified RNA oligonucleotides comprising 50 nucleotides or more, shorter chemically modified RNA oligonucloetides can be combined via enzymatic ligation to obtain longer RNA oligonucleotides. However, the efficiency of such ligations is also restricted to a small range of modified substrates. Therefore, the inefficient and poor condensation yield, and limited substrate tolerance of ligases also imposes severe limitations to synthesizing long site-specifically modified RNA molecules and, thus, can only be achieved with a lot of effort and costs.

In comparison, the incorporation of chemical modifications or rather functionalization of RNA oligonucleotides during their enzymatic synthesis or synthesis in the organism is even more difficult. The substrate specificity of the enzymes that are used for the RNA oligonucleotide synthesis constitutes a first problem, so that only a small range of modifications are tolerated. This includes for example the non-specific (random) incorporation of chemical modifications via *in vitro* transcription with the T7 RNA polymerase (Langer P.R. et al., Proc. Natl. Acad. Sci., 1981, 78:6633-6637). Furthermore, the site-specific terminal and internal labelling, i.e. the incorporation of site-specific terminal and internal chemical modifications is often not possible, even if a modification is accepted by the enzyme.

Thus, the post-synthetic incorporation of chemical modifications or rather functionalization of RNA oligonucleotides can be a valuable alternative to target large and natural RNA oligonucleotide sequences.

There are several methods in the prior art for the post-synthetic enzymatic incorporation of chemical modifications or rather functionalization of RNA oligonucleotides, wherein non-specific, 5'-terminus-specific and 3'-terminus-specific methods are disclosed. For example, Czworkowski J. et al. (Biochemistry, 1991, 30:4821-4830) describe that the 5'-terminus of RNA can be modified by polynucleotide kinases. However, this method has the following disadvantages: The substrate specificity of polynucleotide kinases only allows for chemically very closely related analogs of phosphate, which basically limits the method's applicability to the addition of thiophosphates that can be further reacted with thiol-reactive groups. Furthermore, these reactions are not bioorthogonal (e.g., thiol groups are present in cells and cell extracts). In addition to this, the reagents are less stable in aqueous media, compared to, e.g. azides and alkynes, which may also limit the method's applicability. Moreover, the 5'-termini of RNA sequences within natural isolates usually contain either a cap structure, a tri- or a -monophosphate, which would first have to be removed, resulting in a higher workload. Thus, the present invention particularly relates to methods of post-synthetic incorporation of chemical modifications or rather functionalization of RNA oligonucleotides at their 3'-terminus.

At present, there are four possibilities in order to specifically modify RNA oligonucleotides at the 3'-terminus with post-synthetic enzymatic methods, which are based on the oxidation of 3'-terminal vicinal diols by periodate, the use of T4-RNA-ligase, terminal deoxynucleotidyl transferase (TdT) or poly(A) polymerase (PAP), respectively.

Initially, it is frequently advantageous to modify the 3'-terminus of RNA oligonucleotides by periodate oxidation and subsequent reaction with an amine, carbazide or rather hydrazide, which has been described by Hannske, F. & F. Cramer (Meth. Enzymology, 1979, 59:172-181) for RNA. However, the scope of the 3'-terminus specific labelling based on periodate oxidation is limited because the periodate oxidation and subsequent reaction with e.g. hydrazides blocks the 3'-terminus so that it is not available for further enzymatic reactions anymore.

The second method is based on the enzyme T4-RNA-Ligase that incorporates cytosine-5'3'-diphosphate (pCp)-derivatives or short modified RNA oligonucleotides. However, this method either blocks the 3'-terminus so that it is not available for further enzymatic reactions anymore or bears the risk of multiple incorporations. Thus, the quantification of fluorescence signals is not possible and the sequence is altered in an undefined manner. Furthermore there is the further problem of low efficiency.

Moreover, the specific labelling of RNA oligonucleotides at the 3'-terminus can be achieved by the enzyme TdT (Rosenmeyer V. et al., Anal. Biochem., 1995, 224:446-449) that can attach one or more modified nucleotides to the 3'-terminus. However, the enzymatic incorporation of chemical modifications or rather functionalization of RNA oligonucleotides by TdT has a low efficiency because of its high specificity to deoxyribonucleic acid (DNA), whereas RNA is barely accepted as a substrate.

The fourth method consists in the use of PAP which has been reported by Martin G. & Keller W. (RNA, 1998, 4:226-230) for RNA oligonucleotides. However, direct oligonucleotide labelling with the enzyme PAP is limited to a certain range of modifications because PAP only accepts a small range of modified nucleoside triphosphates (NTP). Thus, the efficiency of labelling depends on the NTP and, thus, the usability of this method is very limited. Furthermore, this method can restrict subsequent enzymatic reactions.

### Problem of the invention

Since the aforementioned technologies have several disadvantages for the post-synthetic enzymatic incorporation of chemical modifications or rather functionalization of RNA oligonucleotides for their labelling, there is a further need for a method, which solves the technical problems as described above.

In summary:
- In the known methods, the enzymatic and chemical reactions involve a disproportionately high amount of effort. Thus, one object of the present invention is the provision of a fast method with a high yield.
- Furthermore, in the known methods, the functional groups are directly attached to the NTPs. These additional functional groups, however, can inhibit the enzymatic incorporation because the modified NTPs are not recognized by the enzyme which causes a very limited number of possible chemical modifications or rather functionalizations. Thus, a further object of the present invention is the provision of a method showing an increased number of possible chemical modifications or rather functionalizations.
- Furthermore, some of the methods of the prior art do not work reliably. Thus, a further object of the present invention is the provision of a method that works reliably.
- In addition, the current methods of the prior art do not provide a method for the enzymatic incorporation of site-specific terminal and internal chemical modifications. Thus, a further object of the present invention is the provision of a method for targeting any position within an RNA oligonucleotide, i.e. a method for the enzymatic incorporation of site-specific terminal and internal chemical modifications or rather functionalization of RNA oligonucleotides.
- Moreover, the methods of the prior art have a low dynamic range only so that they are not suitable for a wide range of applications. Thus, a further object of the present invention is the provision of a method that is suitable for a wide range of applications.

In conclusion, the technical problem of the present invention is to provide an improved method of post-synthetic, enzymatic incorporation of chemical modifications or rather functionalization of RNA oligonucleotides for their labelling that can be applied to many different substrates at variable concentrations to create either site-specific terminal or internal chemical modifications of RNA oligonucleotides with a high number of possible functional groups. Possible fields of application should include e.g., *in vitro* studies of RNA structure and dynamics, modification of RNA in order to optimize its properties, as well as RNA imaging.

This technical problem is solved by the subject-matter of claims 1 and 20. Preferred embodiments are the subject-matter of the dependent claims.

### Summary of the invention

The inventors discovered that such an improved method of post-synthetic incorporation of chemical modifications or rather functionalization of RNA oligonucleotides for their labelling may be based on the enzymatic incorporation of a small functional group that is further converted in a subsequent chemical reaction in which the label of choice can be attached.

In detail, the inventors developed a two-step method for the post-synthetic enzymatic incorporation of chemical modifications or rather functionalization of RNA oligonucleotides for their labelling. In a first step, the RNA oligonucleotide is tailed by the incorporation of a convertible azido-modified (N₃-modified) nucleotide at the 3'-terminus in an enzymatic reaction with a nucleotidyl transferase, e.g., the enzyme poly(A) polymerase (PAP) and an N₃-modified nucleoside triphosphate (N₃-modified NTP). In a second step, the functionalization is accomplished with an azide-reactive group, e.g., via a copper-catalyzed 1,3-dipolar cycloaddition reaction between the incorporated N₃-modified nucleotide and a functional group containing a terminal alkyne moiety (copper-catalyzed click reaction), via a strain-promoted [3 + 2] dipolar cycloaddition between the incorporated N₃-modified nucleotide and a functional group containing a cyclooctyne moiety (strain-promoted click reaction), or via an azide-phosphine conjugation between the incorporated N₃-modified nucleotide and a functional group containing a phosphine moiety (Staudinger ligation). To achieve internal chemical modifications or rather functionalization of RNA oligonucleotides, an additional step can be introduced between the first and the second step, in which a second RNA oligonucleotide is enzymatically attached to the N₃-modified nucleotide at the 3'-terminus of the first step, thus converting the 3'-terminal N₃-modified nucleotide into an internal N₃-modified nucleotide that can be further converted in one of the aforementioned reactions.

### Brief description of the figures

Fig. 1 is the analysis of the tailing of RNA by yeast PAP and *E*. *coli* PAP (Fig. 1A), and by TdT (Fig. 1 B) and Cid 1 PUP (Fig. 1C-D) with 7, 6 or rather 5 different NTP analogs via 15% or 18% sequencing PAGE (seqPAGE). (N.R., no reaction).
Fig. 2 shows the analysis for N₃-modified nucleotide incorporation for 8 N₃-ATP (Fig. 2A), 2'-N₃-2'-dGTP and 2'-N₃-2'dCTP (Fig. 2B) with yeast PAP at RNA concentrations between 0.2 µM and 4 µM and varying incubation times.

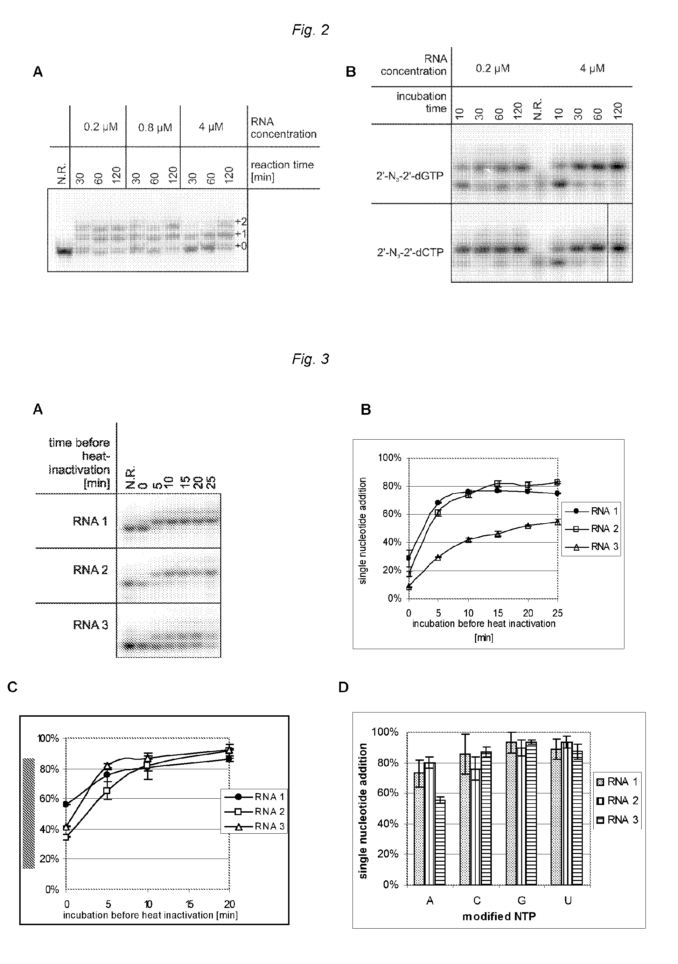

Fig. 3 shows the extent of single N₃-modified nucleotide incorporation. Samples were analyzed by 15% seqPAGE. Bands were quantified using the ImageQuant software. Samples were incubated at 37°C as indicated, followed by 20 min (*E*. *coli* PAP) or 10 min (yeast PAP) of heat inactivation at 65°C. Single N₃-modified nucleotide incorporation was determined in duplicate for each sequence. For all sequences, maximum single N₃-modified nucleotide incorporation efficiency is reached after 20 minutes incubation. All values were determined in triplicate, except for RNA 1, with 2'-N₃-2'-dATP and RNA 3, with 2'-N₃-2'-dGTP (duplicate).
Fig. 3A shows the gel analysis for the maximization of single N₃-modified nucleotide incorporation using *E*. *coli* PAP with 2'-N₃-2'-dATP and 2.5 mM MnCl₂.
Fig. 3B shows the resulting graph for the maximization of single N₃-modified nucleotide incorporation using *E*. *coli* PAP with 2'-N₃-2'-dATP and 2.5 mM MnCl₂.
Fig. 3C shows the maximization of single N₃-modified nucleotide incorporation using yeast PAP with 2'-N₃-2'-dCTP.
Fig. 3D shows the determination of single N₃-modified nuncleotide incorporation efficiency under optimized reaction conditions.
Fig. 4 shows 15% seqPAGE results for the analysis of yeast PAP reactions at elevated RNA oligonucleotide concentrations with 2'-N₃-2'-dCTP and 2'-N₃-2'-dUTP at 4, 20, 40 and 80 µM RNA oligonucleotide concentrations (RNA 2), which were heat-inactivated after the indicated incubation time, respectively, i.e. after 30, 60, or 120 min.
Fig. 5 shows the LC-MS chromatograms (absorption at 254 nm) of non-modified RNA 2 (Fig. 5A) and RNA 2 reacted with 2'-N₃-2'-dCTP and yeast PAP at 80 µM RNA oligonucleotide concentration (Fig. 5B). Deconvoluted masses are given for the major peaks.
Fig. 6 shows the results of incorporation and subsequent click-conversion with Alexa Fluor 647 alkyne for different RNAs.
Fig. 6A shows the results of incorporation and subsequent click-conversion with Alexa Fluor 647 alkyne of all 4 different 2'-N₃-NTPs using the chemically synthesized, radioactively labelled RNA oligonucleotide RNA 1, analyzed by 15% seqPAGE. The upper panel shows radioactivity; the medium panel shows fluorescence (Alexa Fluor 647: ex: 633 nm, em: 670nm BP 30) and the lower panel shows an overlay of both signals with radioactive signal shown in green and fluorescent signals shown in magenta. Presence of both signals results in a bright to white signal.
Fig. 6B shows the determination of the detection limit using the samples from Fig. 6A loaded on a 12% sequencing gel in amounts from 25 amol to 2.5 fmol, scanned for fluorescent signals (Alexa Fluor 647: ex: 633 nm, em: 670nm BP 30) at PMT 900V and high sensitivity.
Fig. 6C shows denaturing PAGE results of 2'-N₃-2'-dUTP incorporation and fluorescent labelling of a total RNA oligonucleotide isolate from *E*. *coli* (23S rRNA and 16S rRNA). Total RNA oligonucleotide isolate was reacted with 2'-N₃-2'-dUTP and yeast PAP, and clicked with Alexa Fluor 647. The overlay (right) shows Sybr-Gold stained RNA (ex: 532 nm, em: 526 nm SP; right lane) and Alexa-Fluor 647 containing RNA (647: ex: 633 nm, em: 670 nm, BP 30; left two lanes). The presence of both results in a bright to white colour.
Fig. 7 shows the LC-MS chromatogram of RNA 2 reacted with yeast PAP and 2'-N₃-2'-dCTP at a RNA concentration of 20 µM and converted with Alexa Fluor 488 alkyne at a RNA concentration of 10 µM. Absorptions at 254 nm (oligonucleotide absorption) and at 492 nm (Alexa Fluor 488 maximum absorption) are given (absorption/mAU), as well as the deconvoluted mass (m_{deconv.}) for the main peak.

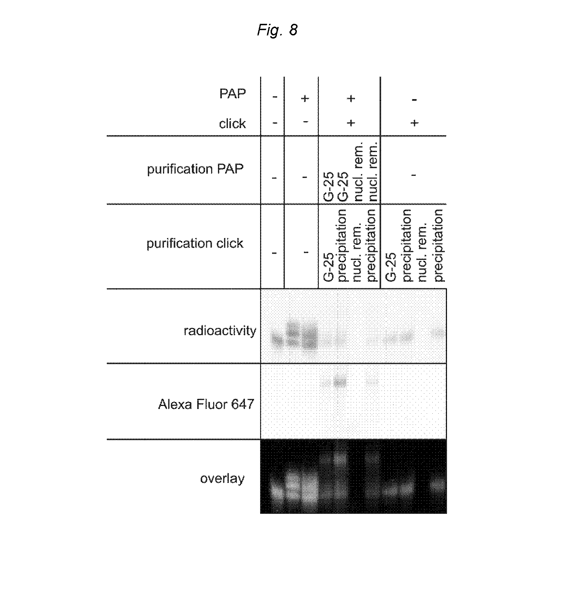

Fig. 8 shows the results of incorporation and subsequent click conversion with Alexa Fluor 647 alkyne of 8-N₃-ATP using RNA 1 and yeast PAP. Different methods were employed to purify both, the PAP reaction (purification PAP) and the click reaction (purification click). PAP reactions were purified either by G-25-spin columns or by QlAquick Nucleotide Removal Kit (nucl. rem.). Click reactions were purified using either the same method as employed for the respective PAP reaction, or ethanol precipitation. The upper panel shows radioactivity; the medium panel shows fluorescence (Alexa Fluor 647: ex: 633 nm, em: 670nm BP 30) and the lower panel shows an overlay of both signals with radioactive signal shown in green and fluorescent signals shown in magenta. Presence of both signals results in a bright to white signal.
   Lane 1 shows unreacted RNA 1, lanes 2 and 3 show PAP reacted RNA 1, lanes 4-7 show PAP reacted and subsequently click reacted RNA 1, purified by various combinations of methods, and lanes 8-11 show RNA 1 that was subjected to click conditions without being reacted with PAP, as a control.
Fig. 9 shows the results of incorporation and subsequent click conversion with biotin alkyne of all four 2'-N₃-2'-dNTPs using RNA 1 and yeast PAP (under suboptimal conditions).
   The left five lanes (1-5) show the result of PAP reaction with the different modified NTPs, the middle four lanes (6-9) show the results of click-conversion with biotin alkyne and the right four lanes (10-13) show the result of click conversion with biotin and subsequent incubation with streptavidin to achieve a streptavidin shift.

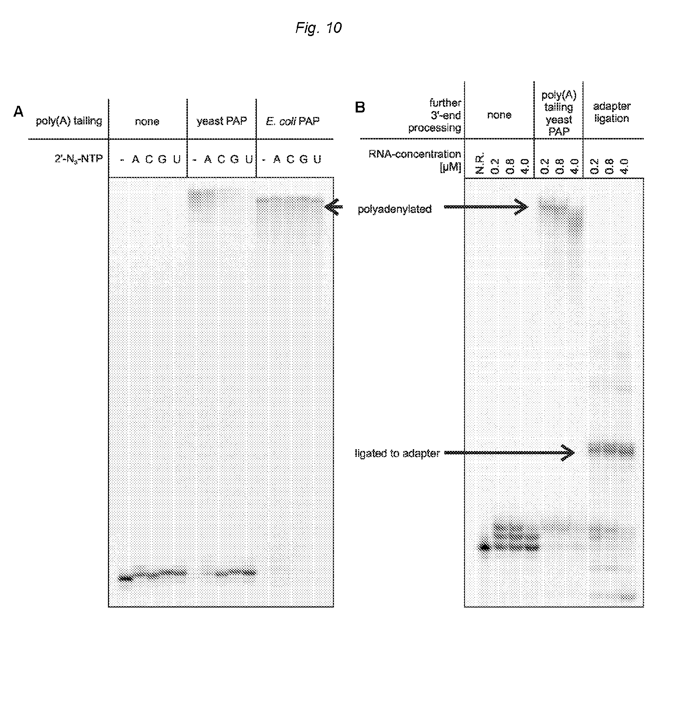

Fig. 10 shows the result of different enzymatic reactions involving the modified 3'-ends of RNA 1 that was previously reacted with yeast or *E*. *coli* PAP (only for 2'-N₃-2'-dATP) and one of the modified NTPs.
Fig. 10A shows ATP-tailing with both different PAPs using as substrate RNA 1 that was previously reacted with yeast PAP or *E*. *coli* PAP (only for 2'-N₃-2'-dATP) and one of the 2'-N₃-2'-dNTPs. The left five lanes (1-5) show the products of the first PAP reaction with modified NTPs. The middle five lanes (6-10) show the products of subsequent ATP-tailing with yeast PAP. The right five lanes (11-15) show the products of subsequent ATP-tailing with *E*. *coli* PAP.
Fig. 10B shows ATP-tailing and adapter ligation of RNA 1 modified with 8-N₃-ATP. RNA 1 at various concentrations (0.2, 0.8, and 4 µM) was first reacted with 8-N₃-ATP (lanes 1-3) and further subjected to either yeast PAP tailing with ATP (lanes 4-6) or adapter ligation (lanes 7-9).

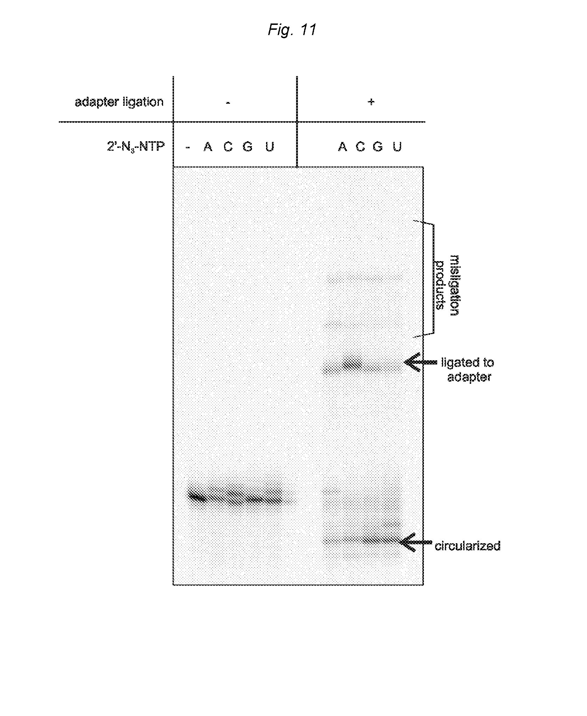

Fig. 11 shows the results of adapter ligation of RNA 1 reacted with all four 2'-N₃-modified NTPs and yeast PAP (under suboptimal conditions). Lanes 1-5 show the results of PAP reactions (-), lanes 6-9 show the results of subsequent adapter ligations (+).

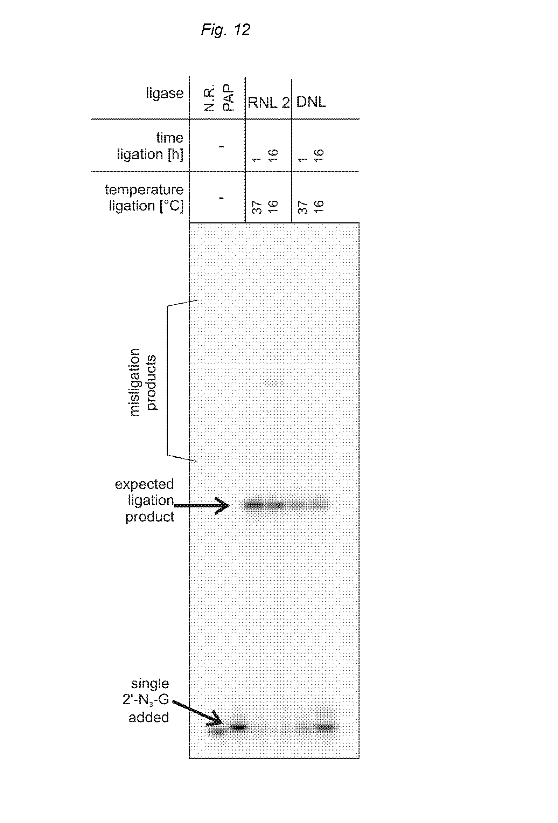

Fig. 12 shows the creation of internal modifications, more specifically the ligation of N₃-modified RNA 1 by T4 DNA ligase (DNL) or T4 RNA ligase 2 (RNL 2).

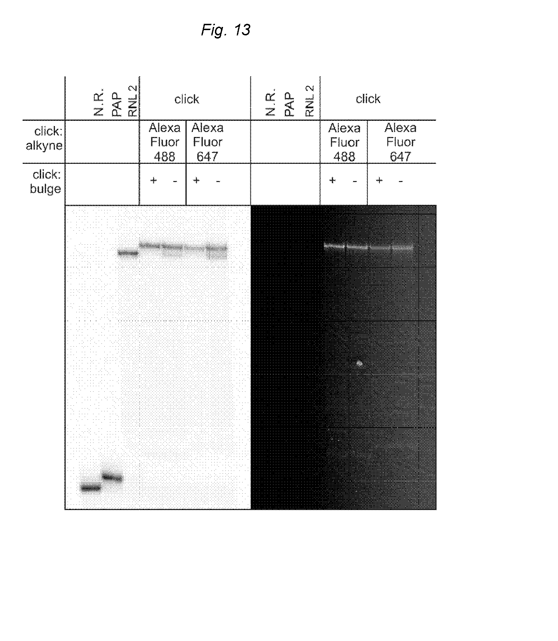

Fig. 13 shows the creation of internal modifications, more specifically the click reaction of Alexa Fluor 488 or Alexa Fluor 647 alkyne and internal azide with and without formation of a bulge-loop. Radioactive scan (left) and fluorescence scan (right); middle two lanes of the fluorescence scan, shown in green: Alexa Fluor 488, ex: 488 nm, em: 520 nm BP 40, right two lanes of the fluorescence scan, shown in magenta: Alexa Fluor 647, ex: 633 nm, em: 670 nm BP 30.

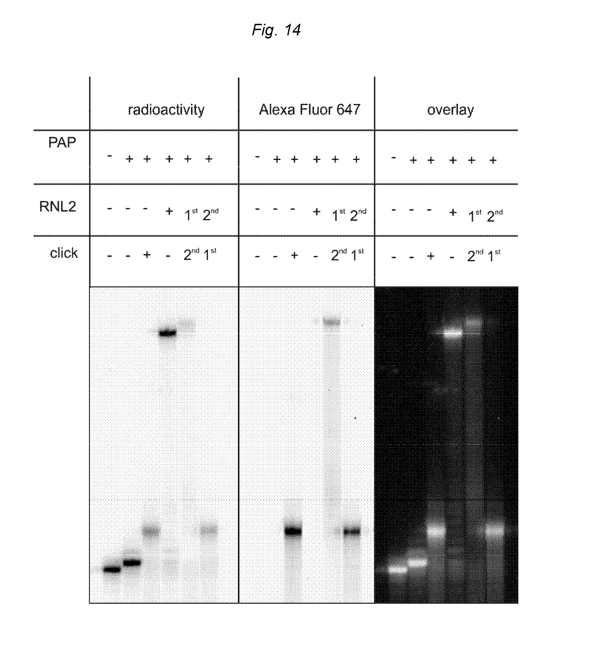

Fig. 14 shows the results of incorporation of 2'-N₃-2'-dGTP followed by ligation and then click reaction or click reaction and then ligation. The left panel shows radioactivity, i.e. the storage phosphor screen scan, the middle panel shows fluorescence, i.e. Alexa Fluor 647 scan (ex: 633 nm, em: 670 nm BP 30), and the right panel shows the overlay of both signals with radioactive signal shown in green and fluorescent signals shown in magenta. Presence of both results in bright to white signals.

### Detailed description of the invention

The present invention concerns a method for the functionalization of RNA oligonucleotides, particularly for their labelling, by enzymatically incorporating a convertible azido-modified (N₃-modified) nucleotide in a first step, which is in a second step conjugated with an azide-reactive group, e.g., with a terminal alkyne moiety by a copper-catalyzed click reaction or with a cyclooctyne moiety by a strain-promoted click reaction, or with a phosphine by Staudinger ligation, wherein this 3'-terminal chemical modification can be converted into an internal chemical modification in an additional step between the first and the second step.

"Functionalization" in general is the addition of functional groups into a molecule or onto the surface of a material. Functionalization of the method of the present invention is on the one hand the incorporation of a convertible N₃-modified nucleotide at the 3'-terminus of an RNA oligonucleotide in an enzymatic reaction with a nucleotidyl transferase, e.g., the enzyme PAP and an N₃-modified NTP (first step) and on the other hand the reaction between the incorporated N₃-modified nucleotide with a functional group containing, e.g., a terminal alkyne moiety, a cyclooctyne moiety, e.g., a strained cyclooctyne moiety, or a phosphine moiety, e.g., an ester-derivatized phosphine-moiety (second step).

"Functional groups" in general are specific groups of atoms within molecules that are responsible for the characteristic chemical reactions of those molecules. The functional groups of the present invention can be selected from the group ranging from various fluorophores to affinity-tags like biotin, sugars, lipids, sterols, PEG-linkers, co-factors that may be used to influence RNA properties like solubility, membrane permeability, and localization, and various reactive moieties that permit crosslinking of RNA to proteins, which can be used to study RNA-protein interactions, radioactive or electrochemical labels. In the method of the present invention, the functional groups contain an azide-reactive group, e.g., a terminal alkyne moiety, a cyclooctyne moiety, or phosphine moiety. In a preferable embodiment, the terminal alkyne moiety is selected from the group of consisting of aliphatic and aromatic terminal alkynes, e.g., Alexa Fluor 488 alkyne, Alexa Fluor 647 alkyne and biotin-alkyne; the cyclooctyne moiety is a strained cyclooctyne moiety that is selected from the group consisting of carbocyclic or heterocyclic cyclooctynes that may or may not be fused with aromatic moieties and may or may not contain favourable substituents like fluor or methoxy, influencing reaction rates or molecular properties, e.g., azacylcooctyne, benzocyclooctyne, dibenzocyclooctyne, and difluorocyclooctyne, modified with various labels, e.g., Dibenzylcyclooctyne-Fluor 488, Dibenzylcyclooctyne-Fluor 525, Dibenzylcyclooctyne-Fluor 585, or Dibenzylcyclooctyne-Biotin Conjugate; and the phosphine moiety is an ester-derivatized phosphine moiety that is selected from the group consisting of suitably substituted alkyl- and or triarylphosphines, e.g., triphenylphosphine, 2,2'-bis(diphenylphosphino)-1,1'-binaphthyl and triisopropylphosphine modified with various labels, e.g., DyLight 488-Phosphine, DyLight 549-Phosphine, or DyLight 649-Phosphine.

"Labelling" of the present invention comprises "fluorescent labelling" and "radioactive labelling". Fluorescent labelling is the method of covalently attaching a fluorophore to another molecule, such as a protein or nucleic acid. This is generally accomplished using a reactive derivative of the fluorophore that selectively binds to or reacts with the "functional group" present in the target molecule. In radioactive labelling, radioactive labels, i.e. substances containing a radioisotope are used to measure the speed of chemical processes and to track the movement of a substance. A number of different radioactive forms of hydrogen, carbon, phosphorus, sulfur, and iodine can be used in the present invention, including tritium, carbon-11, carbon-14, phosphorous-32, phosphorous-33, sulphur-35, iodine-123 and iodine-125. According to the present invention, RNA oligonucloetides are labelled by functionalization that refers on the one hand to the incorporation of a convertible N₃-modified nucleotide at the 3'-terminus of a RNA oligonucleotide in an enzymatic reaction with a nucleotidyl transferase, e.g., the enzyme PAP and an N₃-modified NTP (first step) and on the other hand to the reaction between the incorporated N₃-modified nucleotide with a functional group containing, e.g., a terminal alkyne moiety, a strained cyclooctyne moiety or an ester-derivatized phosphine-moiety (second step).

"Tailing" of the present invention means the incorporation of a convertible N₃-modified nucleotide at the 3'-terminus of a RNA oligonucleotide in an enzymatic reaction with a nucleotidyl transferase, e.g., the enzyme PAP and an N₃-modified NTP (first step).

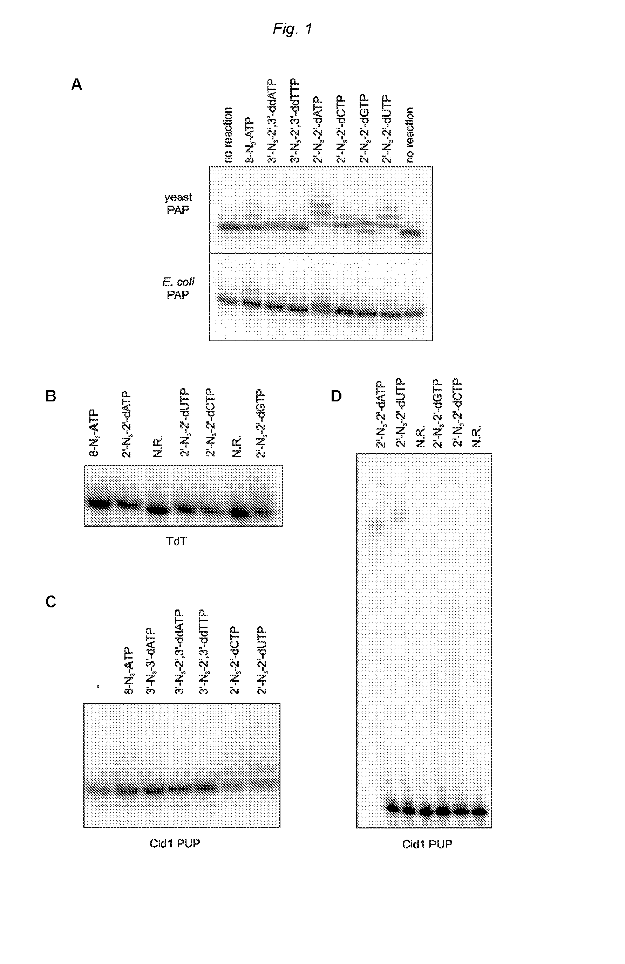

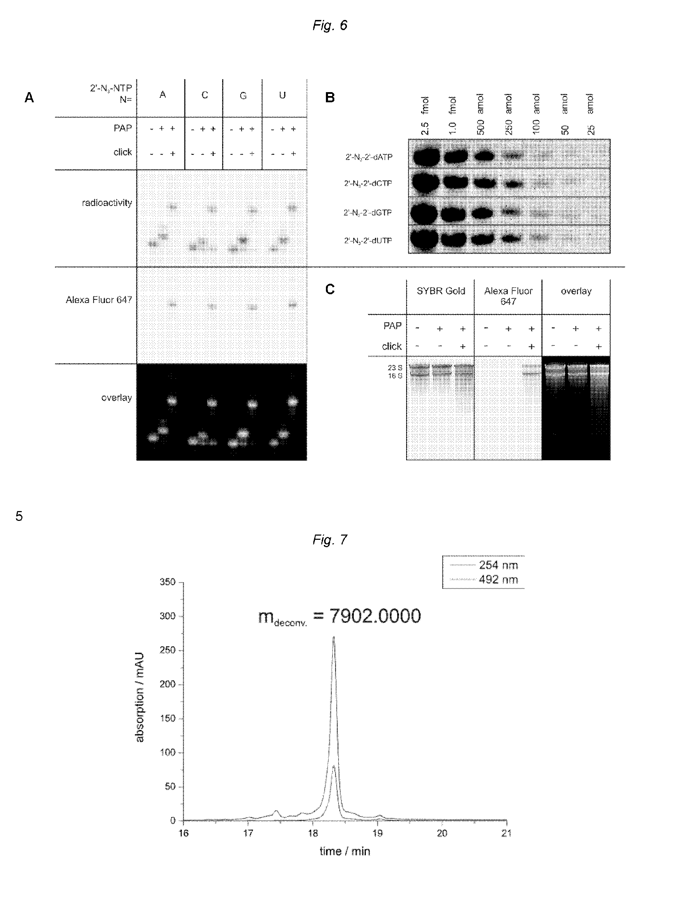

"RNA oligonucleotides" of the present invention are nucleic acid polymers of any length that do not underlie any restrictions as regards to their sequences. They can be achieved via chemical synthesis, enzymatic synthesis or synthesis in the organism, where the synthesized RNA oligonucleotides are directly derived from the organism. In a preferable embodiment, chemically synthesized RNA oligonucleotides are used (Fig. 1, 2, 4, 6A). In a preferable embodiment, chemically synthesized, or in vitro synthesized RNA oligonucleotides with fifty or more bases are used. In another preferable embodiment, chemically synthesized RNA oligonucleotides with fifty or fewer bases are used. In yet another preferable embodiment, RNA oligonucleotides isolated from a living organism (Fig. 6C) of any length are used.

The method for the functionalization of RNA oligonucleotides of the present invention is based on post-synthetic, enzymatic incorporating a convertible azide (N₃) modified NTP (N₃-modified NTP) in a first step, wherein this enzymatic reaction is preferably carried out by nucleotidyl transferases, which are selected from the group consisting of yeast PAP, *Escherichia coli* PAP (*E. coli* PAP), *Schizosaccharomyces pombe* cytoplasmic protein Cid 1 that acts as a poly(U) polymerase (Cid 1 PUP) and TdT. In a more preferable embodiment, the enzymatic reaction is carried out by yeast PAP or *E. coli* PAP.

"Azide" (N₃) of the present invention refers to organic azide with a linear structure and can be drawn, according to the valence bond theory, as different resonating structures.

"Azido-modified NTP" (N₃-modified NTP) refers to an azido-modified nucleoside triphosphate, wherein a nucleoside triphosphate is a nucleoside with three phosphates. Natural nucleoside triphosphates include adenosine triphosphate (ATP), guanosine triphosphate (GTP), cytidine triphosphate (CTP), thymidine triphosphate (TTP) and uridine triphosphate (UTP). These terms refer to those nucleoside triphosphates that contain ribose.

The nucleotidyl transferases of the present invention, however, exhibit an incorporation activity for the whole spectrum of N₃-modified NTPs, which are selected from the group consisting of adenosine triphosphate (ATP), guanosine triphosphate (GTP), cytidine triphosphate (CTP), thymidine triphosphate (TTP), and uridine triphosphate (UTP); deoxyadenosine triphosphate (dATP), deoxyguanosine triphosphate (dGTP), deoxycytidine triphosphate (dCTP), deoxythymidine triphosphate (dTTP), and deoxyuridine triphosphate (dUTP); dideoxyadenosine triphosphate (ddATP), dideoxyguanosine triphosphate (ddGTP), dideoxycytidine triphosphate (ddCTP), dideoxythymidine triphosphate (ddTTP), and dideoxyuridine triphosphate (ddUTP), wherein the nucleotides containing TTP can also be used for the functionalization of RNA oligonucleotides because they produce the same results as the nucleotides containing UTP. NTPs modified at any position with an N₃-modified can be used. In a preferable embodiment, NTPs modified at the C-8 position, the C-2', or the C-3' position are used (Fig. 1, Fig. 2, and Fig. 3), more preferably for 8-N₃-NTP, 2'-N₃-2'-dNTP, 3'-N₃-3'-dNTP or 3'-N₃-2',3'-ddNTP, even more preferably 8-N₃-ATP, 2'- N₃-2'-dATP, 2'- N₃-2'-dCTP, 2'- N₃-2'-dGTP, 2'- N₃-2'-dUTP, 3'- N₃-2',3'-ddATP, 3'- N₃-2',3'-ddTTP, or 3'-N₃-3'-dATP, most preferably 8-N₃-ATP or 2'-N₃-2'-dATP (Table 1).

**Table 1**

| | | | |
|---|---|---|---|
| 1 | 8-N₃-ATP | (Na-salt) | BIOLOG Germany |
| 2 | 2'- N₃-2'-dATP | (Li-salt) | tebu-bio Germany |
| 3 | 2'- N₃-2'-dCTP | (Na-salt) | IBA GmbH |
| 4 | 2'- N₃-2'-dCTP | (Li-salt) | tebu-bio Germany |
| 5 | 2'- N₃-2'-dGTP | (Li-salt) | tebu-bio Germany |
| 6 | 2'- N₃-2'-dUTP | (Na-salt) | IBA GmbH |
| 7 | 2'- N₃-2'-dUTP | (Li-salt) | tebu-bio Germany |
| 6 | 3'- N₃-2',3'-ddATP | (Na-salt) | IBA GmbH |
| 7 | 3'- N₃-2',3'-ddTTP | (Na-salt) | IBA GmbH |
| 8 | 3'- N₃-3'-dATP | (Na-salt) | IBA GmbH |

After incorporation of the N₃-modified nucleotide, the N₃-modified NTP is generally converted into an N₃-modified NMP, wherein NMP is a nucleoside with one phosphate group, since pyrophosphate is cleaved off the NTP in the course of the enzymatic reaction. N₃-modified nucleotide or N₃-modified nucleoside in this invention can describe both, the N₃-modified NTP before incorporation, or the N₃-modified NMP after incorporation. The term is usually used to describe the NMP.

In a preferable embodiment, the nucleotidyl transferases incorporate either one or more than one N₃-modified nucleotide to the RNA oligonucleotide (single or multiple N₃-modified nucleotide incorporation), respectively, more preferably the nucleotidyl transferases incorporate one or two N₃-modified nucleotides, after which further nucleotide additions do not occur so that the length of the RNA is minimally altered, i.e. relatively short tails are generated by e.g., yeast PAP with 2'-N₃-2'-dCTP, -GTP and -UTP (Fig. 1, Fig. 2, Fig. 3). In a more preferable embodiment, the nucleotidyl transferase adds only one N₃-modified nucleotide to the RNA, which can be achieved by controlling the reaction time.

According to the present invention, the reaction time of the first step varies between 1 min and 18 h, wherein in a preferable embodiment, the reaction time is 5-120 min, more preferably 5, 10, 20, 30, 60, 80 or 120 min, most preferably 30, 60 or 120 min. Extending the reaction time leads to an increase of multiple N₃-modified nucleotide incorporation and thereby to a reduction of RNA products containing only one N₃-modified nucleotide. In a preferable embodiment of the present invention, at least one N₃-modified nucleotide is incorporated after at least 5-10 min and multiple N₃-modified nucleotides are incorporated after at least 30 min, wherein in one case, the optimal reaction time varies from NTP to NTP and in another case, the optimal reaction time varies from NTP to NTP and depends on the enzyme heat inactivation time and temperature, and in a further case, the optimal reaction time varies from NTP to NTP and depends on the removal of the enzyme by an extraction method or by oligonucleotide precipitation. In the first case, the enzymatic reaction is not stopped by heat inactivation so that the optimal reaction time for the incorporation of only one N₃-modified NTP to the RNA oligonucleotide is between 1 min and 18 h, preferably between 5-120 min, even more preferably 30, 60 or 120 min. In the second case, where the enzymatic reaction is stopped by heat inactivation, the optimal reaction time for the incorporation of only one N₃-modified NTP to the RNA oligonucleotide is between 1 min and 18 h, more preferably between 5-120 min, even more preferably 5, 20 or 120 min, most preferably 5 min for 2'-N₃-2'-dUTP, 20 min for 2'-N₃-2'-dCTP (Fig. 3C), 20 min for 2'-N₃-2'-dATP (Fig. 3A and 3B), and 120 min for 2'-N₃-2'-dGTP. In this case, the time and temperature for heat inactivation varies between 5-30 min and 60-100°C, more preferably 10 or 20 min and 65 or 80°C, most preferably 10 min and 65°C using yeast PAP and 20 min and 65°C using *E*. *coli* PAP. In the third case, where the enzymatic reaction is stopped by the removal of the enzyme, the optimal reaction time for the incorporation of only one N₃-modified NTP to the RNA oligonucleotide is between 1 min and 18 h, more preferably between 5-120 min, even more preferably 5, 20 or 120 min, most preferably 5 min for 2'-N₃-2'-dUTP, 20 min for 2'-N₃-2'-dCTP, 20 min for 2'-N₃-2'-dATP, and 120 min for 2'-N₃-2'-dGTP.

In another embodiment of the present invention, the enzymatic action of the first step can be facilitated by the addition of salts, which means that the reaction time of the first step can be decreased by the addition of salts to the reaction buffer so that the nucleotidyl transferase efficiency is increased, preferably between 2-10-fold, more preferably 5-fold. In a preferable embodiment, MnCl₂ is added to the reaction buffer, more preferably between 1-5 mM MnCl₂, even more preferably 2.5 mM MnCl₂. In another preferable embodiment, the salt is added to a reaction sample together with yeast PAP and *E*. *coli* PAP, more preferably to a reaction sample with *E*. *coli* PAP (Fig. 3A, 3B and 3D).

In a further embodiment of the present invention, the enzymatic action of the first step can be facilitated by the use of a partially complementary oligonucleotide that positions the reactive center in a sterically accessible conformation so that the nucleotidyl transferase efficiency is increased.

According to the present invention, the amount of RNA oligonucleotides to be modified in the first step varies considerably depending on the experimental setup. While samples from natural sources can potentially be scarce, highly abundant natural RNAs or chemically synthesized sequences are available at high concentrations. In a preferable embodiment of the method of this invention, the RNA oligonucleotide concentration varies depending on the reaction time and/or N₃-modified NTP species, and is adjusted between 0.05-500 µM, preferably between 0.2-80 µM, more preferably 0.2, 4, 20, 40 and 80 µM, even more preferably between 0.2-4 µM, most preferably 0.2 or 4 µM (Fig. 2).

In another preferable embodiment, the reaction samples are incubated at 20, 25, 37 and 40°C, more preferably at 37°C in the first step, wherein in one case, the enzymatic reaction is not stopped by heat inactivation and in another case, the enzymatic reaction is stopped by heat inactivation. The time and temperature for heat inactivation varies between 5-30 min and 60-100°C, more preferably 10 or 20 min and 65 or 80°C, most preferably 10 min and 65°C using yeast PAP and 20 min and 65°C using *E*. *coli* PAP.

Subsequent to the functionalization of RNA oligonucleotides for their labelling by enzymatic incorporation of a convertible N₃-modified nucleotide in a first step, the N₃-modification of the present invention is in a second step conjugated with an azide-reactive group by azide-alkyne-cycloaddition, e.g., with a terminal alkyne moiety employing the copper-catalyzed click reaction or with a cyclooctyne moiety employing the strain-promoted click reaction. Alternatively, the N₃-modification of the present invention is in a second step conjugated with an azide-reactive group by azide-phosphine-conjugation, e.g. with a phosphine moiety employing the Staudinger ligation.

"Click-chemistry" is a term that was introduced by K. B. Sharpless in 2001 in order to describe reactions, i.e. "click reactions" that are usually bioorthogonal, high-yielding, and occur under relatively mild conditions (Best, M., Biochemistry, 2009, 48:6571-6584). They are wide in scope, create only byproducts that can be removed without chromatography, are stereospecific, simple to perform, and can be conducted in easily removable or benign solvents. This concept was developed in parallel with the interest within the pharmaceutical, materials, and other industries in capabilities for generating large libraries of compounds for screening in discovery research. Several types of reaction have been identified that fulfill these criteria, thermodynamically-favored reactions that lead specifically to one product, such as nucleophilic ring opening reactions of epoxides and aziridines, non-aldol type carbonyl reactions, such as formation of hydrazones and heterocycles, additions to carbon-carbon multiple bonds, such oxidative formation of epoxides and Michael Additions, and cycloaddition reactions.

In the method of the present invention, the term "click reaction" means any reaction that falls into the definition of click reaction by Barry Sharpless and that involves an azide as reactive moiety. The "azide-alkyne-cycloaddition" (cycloaddition) can be employed either copper-catalyzed or copper-free (metal-free), in case of the "strain-promoted click reaction". In case of the copper-catalyzed click reaction, a 1,3-dipolar azide-alkyne cycloaddition is employed. In case of the strain-promoted click reaction, a strain-promoted [3 + 2] dipolar azide-alkyne cycloaddition is employed.

In detail, "copper-catalyzed click reaction" (Azide-Alkyne Huisgen Cycloaddition) means the copper-catalyzed 1,3-dipolar azide-alkyne-cycloaddition, i.e. a 1,3-dipolar cycloaddition between an azide and a terminal alkyne to give a 1,2,3-triazole such that in one preferable embodiment of the present invention the functionalization is accomplished via a copper-catalyzed 1,3-dipolar cycloaddition reaction between the incorporated N₃-modified nucleotide and a functional group containing a terminal alkyne moiety in the second step. Rolf Huisgen was the first to understand the scope of this organic reaction (Huisgen, R., Proc. Chem. Soc. London, 1961, 357). Here, the reactive groups needed (terminal alkynes and azides) are particularly small. Thus, they constitute only minor changes to the overall structure of nucleic acids modified with those groups, as well as the monomers needed to build up the RNA oligonucleotides, so that enzymatic incorporation is more probable. As a result, a high number of different labels containing the matching reactive group can be incorporated in a subsequent chemical reaction. For example, the copper-catalyzed click reaction can be used to attach fluorophore azides to an enzymatically incorporated alkyne. However, instead of incorporating an alkyne into the oligonucleotide, it is also possible to incorporate an azide.

The copper-catalyzed click reaction according to the present invention is employed using copper(I) (Cu(I)) or a mixture of copper(II) (Cu(II)) with a reducing agent to produce Cu(I) *in situ,* wherein the use of Cu(II) with a reducing agent is preferred. The Cu(I) species of the present invention can be derived from commercial sources of Cu(I), which are selected from the group consisting of cuprous bromide or cuprous iodide. Furthermore, according to the present invention, Cu(II) species can be derived from Cu(II) sulfate, wherein the reducing agent can be sodium ascorbate, ascorbic acid or other. The copper-catalyzed click reaction of the present invention can be run in a variety of solvents and mixtures of water and a variety of partially miscible organic solvents including alcohols, dimethyl sulfoxide (DMSO), dimethylformamide (DMF), tert-butanol (tButOH) and acetone. As Cu(I) is unstable in aqueous solvents, stabilizing ligands are effective for improving the reaction outcome of the present invention, wherein tris-(benzyltriazolylmethyl)amine (TBTA) or tris-(3-hydroxypropyltriazolylmethyl)amine (THPTA) is preferably used, more preferably THPTA (Fig. 6-9).

Though the ligand does not have a direct influence on the integrity of the reaction product, the presence of a ligand has its advantages in the present invention. The ligand prevents the copper-ion from interacting with the RNA oligonucleotide leading to degradation and formation of side products and also helps keeping the level of other harmful reaction products low. Furthermore, the ligand can potentially function as a proton acceptor thus eliminating the need of an additional base. Following, in a preferable embodiment of the present invention, the copper-catalyzed click reaction can be carried out in presence of a ligand and, thus, in absence of a base. This is advantageous, since the presence of an additional base can lead to increased RNA degradation.

Furthermore, the "strain-promoted click reaction" means in detail a strain-promoted [3 + 2] dipolar azide-alkyne cycloaddition leading to the formation of a triazole (van Delft, P. et al., Org. Lett. 2010, 12:5486-5489 and Jayaprakash, K.N., et al., Org. Lett, 2010, 12:5410-5413) such that in another preferable embodiment of the present invention the functionalization is accomplished via a strain-promoted [3 + 2] dipolar cycloaddition between the incorporated N₃-modified nucleotide and a functional group containing a cyclooctyne moiety or rather a strained cyclooctyne moiety in the second step.

Moreover, another click reaction involving an azide-reactive group is the "Staudinger ligation" (azide-phosphine conjugation), which represents a modification of the classical Staudinger reaction in which phopshine-activated proteins or labelling reagents react with azide-labeled target molecules to form aza-ylide intermediates that quickly rearrange in aqueous conditions to form stable amide bonds between reactant molecules (Saxon, E. & Bertozzi, C.R., Science, 2010, 287:2007-2010). Thus, in a further preferable embodiment of the present invention, the functionalization is accomplished via an azide-phosphine conjugation between the incorporated N₃-modified nucleotide and a functional group containing a phosphine moiety or rather an ester-drivatized phosphine-moiety in the second step. In comparison to the copper-catalyzed click reactions, the strain-promoted click reaction and the Staudinger ligation are more compatible with living cells and will therefore allow for new *in vivo* applications.

Furthermore, another preferable embodiment of the method of the present invention provides the possibility of "splinted ligation" of the RNA oligonuclotides in which the N₃-modified nucleotides are incorporated at the 3'-terminus, wherein the 3'-terminal N₃-modification can be converted into an internal N₃-modification (Fig. 10-14) between the first and the second step, i.e. the present invention provides a method for the enzymatic incorporation of site-specific terminal chemical modifications that can be converted into internal chemical modifications. However, depending on whether or not the internal chemical modification is situated in a base-paired region or in a more flexible loop, it is known that e.g., the copper-catalyzed click reaction can be hampered so that the reaction yields are decreased. Thus, in a more preferable embodiment of the method of the present invention, higher Cu and alkyne concentrations, preferably 1-10-fold higher Cu and alkyne concentrations, more preferably 5-fold higher Cu concentrations and 10-fold higher alkyne concentrations are applied in order to increase the reaction yields of copper-catalyzed click reactions where the azide has been converted into an internal modification, e.g., by splinted ligation (Fig. 13). Further, in another preferable embodiment, a DNA strand with partial reverse complementarity to the ligated sequence is applied in order to increase the reaction yields of copper-catalyzed click reactions where splinted ligation is employed (Fig. 13).

Moreover, other methods can be applied in order to convert the 3'-terminal N₃-modified nucleotide into an internal N₃-modified nucleotide, involving different kinds of ligases that work with or without the help of a splint oligonucleotide, as well as various nucleotidyl transferases that add one or several further nucleotides to the N₃-modified 3'-terminus.

In addition, the present invention concerns a kit for the functionalization of RNA oligonucleotides for their labelling by enzymatic incorporation of a convertible N₃-modified nucleotide in a first step which is in a second step conjugated with an azide-reactive group by a copper-catalyzed click reaction (e.g. with a terminal alkyne moiety), by a strain-promoted click reaction (e.g. with a cyclooctyne moiety), or by Staudinger ligation (e.g. with a phosphine moiety), wherein this 3'-terminal chemical modification can be converted into an internal chemical modification in an additional step between the first and the second step. The preferable embodiments of above can be also applied on the kit.

In summary, the new method of post-synthetic, enzymatic incorporation of chemical modifications or rather functionalization of RNA oligonucleotides for their labelling of the present invention solves the technical problems of the technologies as described in the prior art. In detail, the new method of the present invention represents a fast method with a high yield, shows an increased number of possible chemical modifications or rather functionalizations, works reliably, can be used for targeting any position within an RNA oligonucleotide, i.e. represents a method for the enzymatic incorporation of site-specific terminal and internal chemical modifications or rather functionalization, and is suitable for a wide range of applications.

### Examples

Hereinafter, the present invention is described in more detail and specifically with reference to the Examples, which however are not intended to limit the present invention.

In general, RNA 1 and all DNA-oligonucleotides of Table 2 were purchased from the IBA GmbH (Göttingen, Germany). RNA 2 and RNA 3 were obtained from the Institute of Pharmacy and Biochemistry, Johannes-Gutenberg Universität Mainz, Germany. RNA 4 was purchased from Dharmacon, Inc. (Chicago, US). Modified NTPs were purchased either from BIOLOG (Bremen, Germany), the IBA GmbH or tebu-bio Germany (Offenbach, Germany). Yeast PAP was purchased from USB (High Wycombe, UK). *E.coli* PAP, Cid1 PUP, and RNA ligase 2, truncated were purchased from New England Biolabs (Ipswich, US). T4 polynucleotide kinase, TdT, T4 DNA ligase (DNL), T4 RNA ligase (1) (RNL1) and DNase I were purchased from the Fermentas GmbH (St. Leon-Rot, Germany). T4 RNA ligase 2 (RNL2) was prepared in-house. Alexa Fluor 647 alkyne, Alexa Fluor 488 alkyne, and biotin-alkyne were purchased from the Invitrogen GmbH (Darmstadt, Germany). Tris-(3-Hydroxypropyltriazolylmethyl)amine (THPTA) was synthesized according to the published procedure (Hong, V. et al., Chem. Int. Ed. 48, 2009, 9879 -9883). The streptavidin solution was from the Thermo Fisher Scientific GmbH (Dreieich, Germany). *E*. *coli* cells of strain JM 109 were from Promega (Mannheim, Germany). The Dibenzylcyclooctyne-Fluor 488, Dibenzylcyclooctyne-Fluor 525, Dibenzylcyclooctyne-Fluor 585, or Dibenzylcyclooctyne-Biotin Conjugate were purchased from Jena Bioscience GmbH (Jena, Germany). The DyLight 488-Phosphine, DyLight 549-Phosphine, or DyLight 649-Phosphine were purchased from the Thermo Fischer Scientific GmbH (Dreieich, Germany).

### Example 1: Preparation of radioactively labelled RNA oligonucleotides

For 5'-labelling of RNA, 1 µM RNA, 1 µM [γ-³²P]-ATP (10 µCi/µl, 3000 Ci/mmol; Hartmann Analytic GmbH, Braunschweig, Germany), and 1 u/µl T4 Polynucleotide Kinase in 1x T4 PNK buffer A (50 mM Tris-HCl (pH 7.6 at 25°C), 10 mM MgCl₂, 5 mM DTT, 100 µM spermidine) were used. Labelled oligonucleotides were gel-purified and recovered by ethanol precipitation (RNA 1) or isopropanol precipitation (RNA 2, RNA 3).

### Example 2: Enzymatic incorporation of N₃-modified NTPs to the 3'-terminus of RNA oligonucleotides by yeast and E.coli PAP, Cid 1 PUP and TdT

Five pmol of non-labelled RNA 1, doped with 0.1 to 1 pmol of 5'-labelled RNA 1 were incubated with 500 µM of the respective N₃-NTP and the respective nucleotidyl transferase in its recommended buffer at 37°C for 60 or 90 minutes. Amounts of nucleotidyl transferase and buffer used were: 600 units of yeast PAP in 1x USB yeast poly(A) polymerase buffer (20 mM Tris-HCl (pH 7.0 at 25 °C), 0.6 mM MnCl₂, 0.02 mM EDTA, 100 µg/ml acetylated BSA, 10% glycerol) in a final volume of 25 µl; 5 units of *E.coli* PAP in 1x NEB *E*. *coli* poly(A) polymerase buffer (50 mM Tris-HCl (pH 7.9 at 25°C), 250 mM NaCl, 10 mM MgCl₂) in a final volume of 20 µl; 2 units of Cid 1 PUP in 1x NEBuffer 2 (10 mM Tris-HCl (pH 7.9 at 25°C), 50 mM NaCl, 10 mM MgCl₂, 1 mM DTT) in a final volume of 25 µl; 20 units of TdT in 1x Fermentas terminal deoxynucleotidyl transferase buffer (25 mM Tris (pH 7.2 at 25°C), 200 mM potassium cacodylate, 0.01 % (v/v) Triton X-100, 1 mM CoCl₂) in a final volume of 20 µl. Reaction mixtures were analyzed by 15% sequencing PAGE (seqPAGE). To image radioactive bands, the gels were exposed to storage phosphor screens and scanned with a Typhoon 9400 scanner (GE Healthcare, Munich, Germany). For those N₃-modified NTPs that could be efficiently incorporated at the 3'-terminus of the RNA oligonucleotides, the reactions were also performed at a 20-fold higher concentration, employing 100 pmol of non-labelled RNA oligonucleotide, doped with 0.1 or 0.5 pmol of 5'-labelled RNA 1 leaving the reaction conditions and analysis unchanged.

For TdT, the efficiency of N₃-nucleotide addition was found to be very low (Fig. 1 B). Cid 1 PUP accepted 2'-N₃-2'-dNTPs with moderate yield, but not 3'- or C-8 modified NTPs (Fig. 1C). Additionally, whenever this enzyme accepted the modified NTPs, it produced heterogeneous tails (Fig. 1 D). Yeast PAP exhibited the highest incorporation activity for all modified NTPs (Fig. 1A). For those NTPs modified at positions C-2' and C-8, generally 1 to 2 nucleotides were incorporated, except for 2'-N₃-2'-dATP, where 1, 2, 3, and more nucleotides were incorporated. *E. coli* PAP also accepted the whole spectrum of NTPs, but with significantly lower yields (Fig. 1A). Only the incorporation of 2'-N₃-2'dATP proceeded with a good efficiency. Taking together these results, the focus of the present invention was on yeast and *E*. *coli* PAP for more detailed investigations. Results of N₃-nucleotide addition is shown in Fig. 1A for both enzymes.

For the base-modified ATP analog 8-N₃-ATP, both PAP enzymes added either one or two nucleotides to the RNA, after which further nucleotide additions did not occur. Even after two hours of reaction, a considerable fraction of RNA sequences remained unmodified. For yeast PAP, the reaction was less efficient when the RNA concentration increased from 0.2 to 4 µM, and always resulted in a mixture of singly and doubly modified products, irrespective of reaction time and RNA concentration (Fig. 2A). Using 8-N₃-ATP is thus possible to provide RNA sequences with one to two reactive moieties for further click-reaction so that the length of the RNA is only slightly altered.

### Example 3: Determination of optimal conditions for single N₃-modified NTP incorporation

For all four 2'-N₃-NTPs, 100 pmol of RNA 1, RNA 2 and RNA 3, doped with 0.1 or 0.5 pmol of the respective 5' labelled oligonucleotide were incubated with 500 µM 2'-N₃-NTPs and incubated for various times (Fig. 2B for 2'-N₃-2'-dGTP and 2'-N₃-2'-dCTP), with yeast PAP under the reaction conditions mentioned above in a total volume of 25 µl. The enzyme was then heat denatured by incubation at 65°C for 10 minutes. Furthermore, in case of 2'-N₃-2'-dATP, the same experiment was performed with 50 µM and 5 µM 2'-N₃-2'-dATP employing yeast PAP in a total volume of 25 µl, and with 500 µM 2'-N₃-2'-dATP, employing 0.25 or 0.5 units/µl of *E*. *coli* PAP in a total volume of 20 µl. Additionally, the influence of addition of 2.5 mM MnCl₂ on the activity of *E*. *coli* PAP (0.25 units/µl) was evaluated (Fig. 3A) and found to enhance the reaction speed. *E. coli* PAP was heat inactivated by 20 minutes of incubation at 65°C for heat denaturation. In case of 8-N₃-ATP, 5, 20, and 100 pmol of RNA 1, doped with 0.1 pmol of 5' labelled RNA 1 were incubated with 500 µM 8-N₃-ATP and yeast PAP for 30, 60, and 120 minutes (Fig. 2A). The enzyme was not heat-inactivated. Reaction mixtures were analyzed by 15% or 18% denaturing seqPAGE. Band intensities were quantified using the ImageQuant software (GE Healthcare).

In order to control the reaction so as to achieve the addition of a single moiety, the reaction time has been varied between 10 and 120 minutes employing 0.2 and 4 µM RNA concentrations of RNA 1 (Fig. 2B). At 4 µM concentration the overall yield was higher than at 0.2 µM concentration. To compare the efficiencies of N₃-nucleotide addition for different RNA sequences, the same experiment has been carried out for 2 other RNAs (RNA 2, RNA 3) at 4 µM concentration and found that the reaction kinetics were similar. Starting from these results, the optimal reaction time for single nucleotide addition has been determined.

For 2'-N₃-2'-dGTP the highest amount of single nucleotide addition has been observed after 2 hours of reaction time (e.g., Fig. 2B). Extending the reaction time further lead to an increase of multiple nucleotide addition and thereby a reduction of products containing a single N₃-modified NTP. For 2'-N₃-2'-dCTP and 2'-N₃-2'-dUTP, close to all RNA sequences are converted with at least one residue already after 10 minutes, and the extent of multiple nucleotide addition rises after 30 minutes. Therefore, the optimal reaction times were narrowed down to 20 min in case of 2'-N₃-2'-dCTP and to 5 min for 2'-N₃-2'-dUTP. An example for reaction time optimization is shown in Fig. 3C (for 2'-N₃-2'-dCTP). Single nucleotide incorporation efficiencies under optimal reaction conditions are given in Fig. 3D.

Since the use of yeast PAP with 2'-N₃-2'-dATP lead to the addition of multiple moieties, even at low nucleotide concentration and/or short reaction time, *E. coli* PAP seemed to be more suitable to achieve single nucleotide addition in this particular case. To facilitate the reaction, 2.5 mM MnCl₂ has been added to the reaction buffer supplied by the manufacturer (containing 10 mM MgCl₂ but no MnCl₂). MnCl₂ at this concentration has been reported to increase the efficiency of *E*. *coli* PAP by ∼5-fold (Sippel, A. E., Em. J. Biochem., 1973, 37:31-40) and in the present investigation, a considerable acceleration of 2'-N₃-ATP incorporation, so that reaction yields of generally >50% were obtained after 20 min reaction time (Fig. 3A, 3B, 3D). In the absence of MnCl₂, comparable yields were otherwise only achieved after up to 16 hours of incubation at 37°C, which lead to substantial RNA degradation and irreproducible reaction yields.

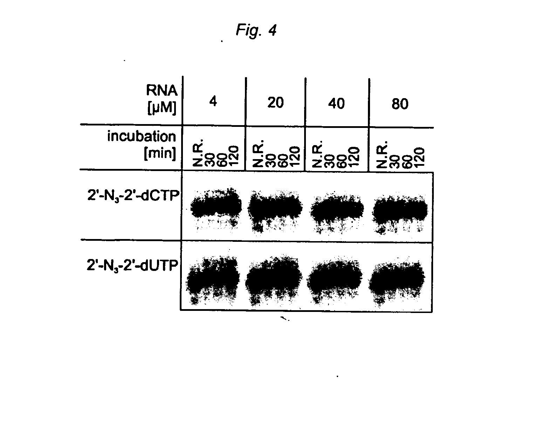

Furthermore, in order to assess the applicability of RNA modification with N₃-modified NTP-analogs by poly(A) polymerase, it has been tested whether incorporation of a single 2'-modified nucleotide is still efficient at high RNA concentrations. Therefore, yeast PAP reaction has been tested with 2'-N₃-2'dCTP and -UTP for RNA 2 at concentrations up to 80 µM and found that efficient addition of a single N₃-modified NTP was possible at prolonged reaction times (Fig. 4).

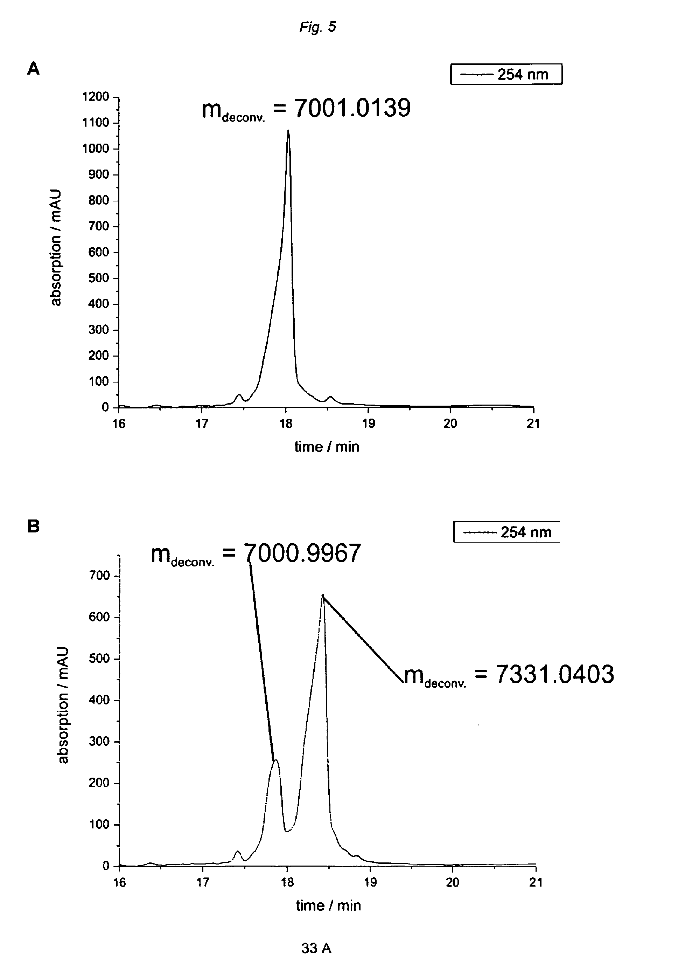

To further confirm the successful incorporation of a single N₃-modified NTP, LC-MS analysis of precipitated product of a reaction containing 80 µM RNA 2 and 2'-N₃-2'dCTP has been performed. In this analysis, a reaction yield of ∼80%, and the identity of the tailing product could be confirmed according to the molecular mass (Fig. 5).

### Example 4: Post-synthetic modification of N₃-modified NTP containing RNA by the copper-catalyzed click reaction

PAP reaction mixtures were purified either by ethanol precipitation (RNA 1), isopropanol precipitation (RNA 2 and RNA 3) or by size exclusion chromatography employing MicroSpin™ G-25 Columns (GE Healthcare), or by using the QIAquick Nucleotide Removal Kit (Qiagen).

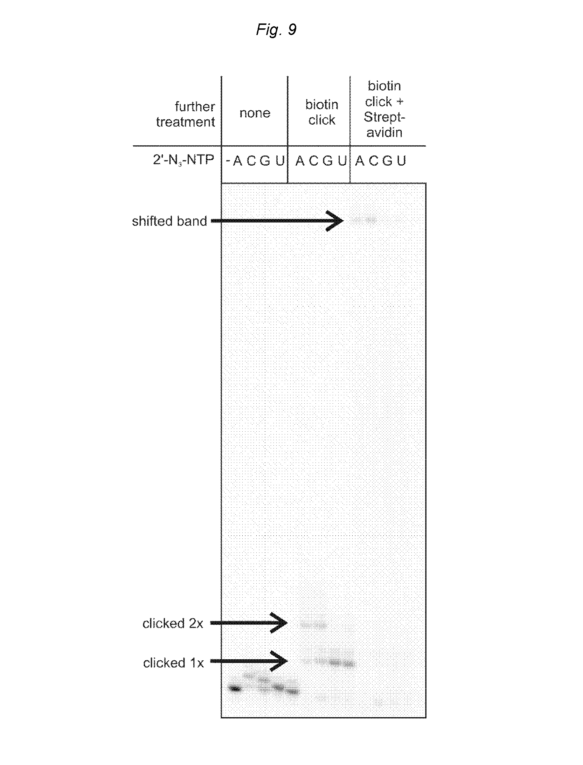

The copper-catalyzed click reaction was performed with an oligonucleotide concentration of 50 nM or 1 µM in 50 mM sodium phosphate buffer (pH 7 at 25°C), 100 µM CuSO₄, 500 µM THPTA, 1 mM sodium ascorbate in a final volume of 10 µl, 50 µl or 100 µl, and varying concentrations of either Alexa Fluor 488 alkyne, Alexa Fluor 647 alkyne or biotin alkyne. Reactions were performed for two hours at 37°C. Reaction mixtures were purified by phenol extraction (only for Alexa Fluor alkynes) and ethanol or isopropanol precipitation and analyzed by 12% or 15% seqPAGE. Bands were visualized by Storage Phosphor Screen scan, as well as fluorescence scan (only for Alexa Fluor alkynes). To compare the different scans, gels were registered using the ImageJ plugin Turboreg (Thévenaz, P. et al., IEEE Transactions on Image Processing, 1998, 7:27-41). In case of Alexa Fluor 647 alkyne, phenol extraction was performed prior to precipitation. For monitoring biotinylation by Streptavidin shift, the oligonucleotides were incubated with Streptavidin solution of at least 5-fold molar excess prior to gel loading (Fig. 9).

RNA modified with any of the four 2'-N₃-NTPs (Fig. 6A) or with 8-N₃-ATP (Fig. 8) could be efficiently conjugated with various functional groups (fluorophores or affinity tags) using the method of the present invention. The resulting product could be detected with an detection limit, so that 100 amol were still visible as a clear band, whereas amounts as low as 25 to 50 amol were still detectable over the background (Fig. 6B). This method has been also applied on chemically synthesized (Fig. 6A) and *in vitro* transcribed RNA oligonucleotides, as well as on total RNA oligonucleotide samples isolated from *E*. *coli* (Fig. 6C). The copper catalyzed click reaction was optimized employing the chemically synthesized RNAs. Purification of the RNA from the PAP reactions by size exclusion chromatography or ethanol precipitation proved to be efficient enough to allow copper catalyzed click reactions with alkyne-modified fluorophores and biotin.

A sample of RNA 2 modified with 2'-N₃-2'-dCTP at a concentration of 20 µM for 2 h with yeast PAP was purified by isopropanol precipitation and subsequently converted with Alexa Fluor 488 alkyne at 10 µM RNA concentration and 500 µM alkyne concentration. The resulting reaction product was analyzed by LC-MS, where a yield of > 98% was confirmed by the UV absorption signal at 254 nm and the identity of the reaction product was confirmed according to the molecular mass (Fig. 7).

### Example 5: Labelling of a natural RNA oligonucleotide isolate

Total RNA oligonucleotide was isolated from *E*. *coli* JM 109 using the GenElute Total RNA Purification Kit (Sigma Aldrich). 28 µg of total RNA (∼ 50 pmol) were converted in a reaction containing 500 µM 2'-N₃-2'dUTP, 600 units of yeast poly(A) polymerase in 1x USB yeast poly(A) polymerase buffer for 30 minutes at 37°C. The enzyme was removed by phenol-ether extraction and the RNA was recovered by ethanol precipitation in presence of Na-acetate.

To perform the click reaction, the resuspended, PAP-reacted total RNA sample (∼1 µM) was incubated with 500 µM Alexa Fluor 647 alkyne, 100 µM CuSO₄, 500 µM THPTA, 1 mM Na-ascorbate in 50 mM sodium phosphate buffer at 25°C for 30 min. RNA was purified directly after the reaction by phenol-ether extraction under addition of 10 mM EDTA and 600 mM Na-acetate, and the RNA oligonucleotide was recovered by ethanol precipitation. About 5 pmol of non-reacted, PAP-reacted, and clicked RNA oligonucleotides were analyzed by 8% denaturing PAGE. (Fig. 6C)

As a result, Fig. 6C shows that the method of the present invention can be also applied to RNA oligonucleotides from a living organism.

### Example 6: 3'-terminal modification of RNA oligonucleotides after PAP reaction with N₃-modified NTPs

To assess whether the 3' termini of RNA oligonucleotides reacted with 8-N₃-ATP or 2'-N₃-2'-dNTPs can still be recognized and modified by different enzymes, various enzymatic reactions were performed with purified reaction products or non-purified, heat inactivated N₃-modified NTP containing PAP reaction mixtures. Polyadenylation of N₃-modified nucleotide-containing, purified RNA by yeast PAP or *E*. *coli* PAP was performed by adding 2.5 pmol of ethanol-precipitated, 2'-N₃-2'-dNTP-reacted RNA into a PAP reaction of 12.5 µl volume (yeast _PAP) or 10 µl volume (*E*. *coli* PAP) as described above, containing 500 µM ATP instead of the modified NTP. Similarly 2 µl of N₃-modified NTP containing yeast PAP mix or 1.6 µl of *E*. *coli* N₃-modified NTP containing PAP mix were added into a reaction mixture as described above, containing 500 µM ATP instead of the modified NTP and adding fresh yeast PAP or *E*. *coli* PAP.

For RNA containing 8-N₃-adenosine at the 3'-terminus (see Fig. 10B), near-quantitative polyadenylation by yeast PAP was observed, especially of those sequences that contained only a single 8-N₃-adenosine moiety. Interestingly, the polyadenylation reaction was inhibited when two 8-N₃-adenosine moieties were present, resulting in measurable amounts of non-polyadenylated sequences. For sequences containing 3'-terminal 2'-N₃-modified nucleotides, the polyadenylation reaction proceeded well when carried out with *E*. *coli* PAP, but was inhibited when yeast PAP was used. (Fig. 10A)

Adapter ligation was performed by adding 2 µl of yeast PAP N₃-NTP containing yeast PAP mix or 1.6 µl of N₃-modified NTP containing *E*. *coli* PAP mix to 7.5 or 10 µM adenylated adapter (DNA 3) prepared according to the published protocol of Hafner, M. et al. (Methods, 2008, 44:3-12). 1 u/µl T4 RNA ligase (1) and 20 u/µl RNA ligase 2, truncated in a final volume of 10 µl ligation buffer (50 mM Tris-HCl pH 7.4, 10 mM MgCl₂). The reactions were incubated at 4 °C overnight.

All reaction mixtures were analyzed by 12% or 15% denaturing sequencing PAGE.

During the analysis of the possibility of ligating adapters to the modified 3'-termini employing T4 RNA ligase 1 and truncated RNA ligase 2 for the addition of preadenylated DNA sequences, a certain preference of non-modified sequences over 8-N₃-A-containing sequences (Fig. 10B) has been observed, but there was no obvious distinction between sequences containing a single or two N₃-modified NTP. For 2'-N₃-modified NTP-containing sequences, such a preference was not observable (Fig. 11). The ligases thus seem to be slightly inhibited by the base-modification but not by the sugar-modification (Fig. 10B, lanes 7-9, Fig. 11).

### Example 7: Splinted ligation of an RNA oligonucleotide to the 3'-terminus of RNA after PAP reaction with N₃-modified NTPs

To convert the 3'-terminal modification into an internal modification, splinted ligation was carried out, employing 0.5 µM purified, 2'- N₃-2'-dGTP-reacted RNA 1, 2.5 µM of RNA 4, 2.25 µM of DNA 1 (splint DNA), and 1.5 units/µl of T4 DNA Ligase or ∼ 500 nmol/µl T4 RNA ligase 2 in 1x ligation buffer (50 mM Tris-HCl (pH 7.4 at 25 °C), 10 mM MgCl₂, 200 µM ATP, and 5 mM DTT), in a final volume of 20 µl. For those samples subjected to copper-catalyzed click reaction, the DTT concentration in the ligase buffer was decreased to 0.5 mM. The mixture was denatured for 30 seconds at 90°C and then cooled to room temperature for 15 minutes prior to addition of the enzyme. The reaction mixture was incubated either at 37°C for 1 hour or at 16°C overnight. The reaction was stopped by heating to 80°C for 10 minutes. The DNA splint was removed by addition of 0.5 units/µl of DNase I (Fermentas) and incubated at 37°C for 15 to 30 minutes, followed by 10 minutes of heat-inactivation at 80°C or phenol-ether extraction (for samples subjected to click reaction). The reaction mixture was purified by ethanol precipitation.

Fig. 12-14 show that splinted ligation is possible with RNA containing an N₃-modified nucleotide, but not when the azide has been converted by click-reaction with an Alexa Fluor 647 alkyne prior to ligation (Fig. 14). Both, T4 DNA ligase and T4 RNA ligase 2 accepted the modified 3'-terminus as substrate. With a 5-fold excess of the second ligation fragment, the T4 DNA ligase achieved ligation yields > 50%, while the ligation was near-quantitative with RNA ligase 2 (Fig. 12). In contrast to this, ligation was not possible any longer when a copper-catalyzed click reaction was performed prior to the ligation. In this case, the modification at the 2'-position of the 3'-terminal nucleotide is much bulkier than just an azide and inhibits the ligase.

### Example 8: Click-reaction of internally N₃-modified RNA oligonucleotides

The copper-catalyzed click reaction with Alexa Fluor 647 alkyne or Alexa Fluor 488 alkyne was carried out as described above or at higher concentrations of alkyne, CuSO₄, THPTA, and Na-ascorbate, and DMSO, and varying the temperature between 37 °C and 50 °C, as indicated. In a different approach, the ligated RNA oligonucleotide was first annealed to a partially reverse complementary DNA that forces the modified position into a 9 nt bulge-loop (DNA 2). In this case, the cycloaddition was carried out with 500 µM CuSO₄, 2.5 mM THPTA and 5 mM Na-ascorbate. The reaction time was two hours. The RNA oligonucleotide was then purified by phenol-ether extraction in presence of 10 mM EDTA and 300 to 600 mM Na-acetate (pH 5.5) followed by ethanol precipitation. Reactions were analyzed by 15% denaturing seqPAGE.

After ligation, the terminal modification is converted to an internal one, so that the accessibility of the azide for both, alkyne reactants, and copper-catalyst is reduced. Depending on whether or not the modified position is situated in a base-paired region or in a more flexible loop, the cycloaddition could be expected to be hampered, which has also been observed for click reactions of 5-ethynyl-deoxyuridine in double-stranded DNA (Wirges, C.T. et al., QSAR Comb. Sci., 2007, 26:1159-1164). Indeed, in the test system of the present invention, where the modified nucleotide is situated in a partially base-paired structure, a decrease in cycloaddition efficiency under standard conditions has been observed. In order to achieve good labelling yields, forced conditions to perform the cycloaddition have been applied. Reaction yields were increased by applying 5-fold higher Cu concentrations and 10-fold higher alkyne-concentrations (Fig. 13). Further increase of reactivity could be achieved by providing a DNA strand with partial reverse complementarity to the ligated sequence that forced the nucleotides 4 nt upstream and 4 nt downstream of the modified nucleotide into a non-paired bulge-loop structure (Fig. 13). Here, reaction yields were surprisingly similar to those observed with 3'-terminal modifications.

Sequence Listing

| | |
|---|---|
| RNA 1 | 5'-GUGACCGCGGAUCGACUUCACCGCGCAGUG-3' |
| RNA 2 | 5'-GCAAGCUGACCCUGAAGUUCAU-3' |
| RNA 3 | 5'-GAACUUCAGGGUCAGCUUGCCG-3' |
| RNA 4 | 5'-phosphate-GCCCGACGAUGCUUCAGCAACCAGUGUAAUGGCG-3' |
| DNA 1 | 5'-CGCCATTACACTGGTTGCTGAAGCATCGTCGGGCCACTGCGCGGTGA AGTCGATCCGCGGTCAC-3' |
| DNA 2 | 5'-CGCCATTACACTGGTTGCTGAAGCATCGTCGCGGTGAAGTCGAT CCGCGGTCAC-3' |
| DNA 3 | Ap-5'-pCTGTAGGCACCATCAAT-3'-block |

## Claims

1. A method for the functionalization of RNA oligonucleotides by enzymatic incorporation of a convertible azido-modified nucleotide in a first step, which is in a second step conjugated with an azide-reactive moiety by a copper-catalyzed click reaction, by a strain-promoted click reaction, or by Staudinger ligation.

2. The method according to claim 1, wherein in the copper-catalyzed click reaction, the azide-reactive moiety is a terminal alkyne moiety, in the strain-promoted click reaction moiety, the azide-reactive moiety is a cyclooctyne moiety, and in the Staudinger ligation, the azide reactive moiety is a phosphine moiety.

3. The method according to claim 1, wherein the enzymatic incorporation occurs post-synthetically.

4. The method according to any of claims 1 to 3, wherein the enzymatic incorporation occurs at the 3'-terminus of the RNA oligonucleotide.

5. The method according to claim 4, wherein the enzymatically incorporated modified nucleotide at the 3'-terminus of the RNA oligonucleotide is converted into an internal modification.

6. The method according to any of claims 1 to 5, wherein the enzymatic incorporation occurs by the enzymatic action of nucleotidyl transferases, including yeast poly(A) polymerase or *Escherichia coli* poly(A) polymerase.

7. The method according to claim 6, wherein the enzymatic action is facilitated by the addition of salts.

8. The method according to claim 6, wherein the enzymatic action is facilitated by the use of a partially complementary oligonucleotide that positions the reactive centre in a sterically accessible conformation.

9. The method according to any of claims 1 to 8, wherein between the first and the second step, a second RNA oligonucleotide is enzymatically attached to the azido-modified nucleotide at the 3'-terminus of the first step.

10. The method according to any of claims 1 to 9, wherein the copper-catalyzed click reaction is a 1,3-dipolar azide-alkyne-cycloaddition.

11. The method according to claim 10, wherein the copper-catalyzed click reaction is employed using copper(I) or a mixture of copper(II) with a reducing agent.

12. The method according to claim 10 or 11, wherein the copper-catalyzed click reaction is carried out in the presence or in the absence of a stabilizing ligand, preferably tris-(benzyltriazolylmethyl)amine or tris-(3-hydroxypropyltriazolylmethyl)amine.

13. The method according to any of claims 10 to 12, wherein the alkyne is selected from the group consisting of aliphatic or aromatic terminal alkynes, preferably Alexa Fluor 488 alkyne, Alexa Fluor 647 alkyne and biotin-alkyne.

14. The method according to any of claims 1 to 9, wherein the strain-promoted click reaction is a strain-promoted [3 + 2] azide-alkyne cycloaddition.

15. The method according to claim 14, wherein the alkyne is a strained cyclooctyne moiety selected from the group consisting of carbocyclic or heterocyclic cyclooctynes that are optionally fused with aromatic moieties and optionally contain fluoro- or methoxy-substituents.

16. The method according to claim 15, wherein the strained cyclooctyne moiety is selected from the group consisting of azacylcooctyne, benzocyclooctyne, dibenzocyclooctyne, and difluorocyclooctyne that are modified with various labels, respectively, preferably Dibenzylcyclooctyne-Fluor 488, Dibenzylcyclooctyne-Fluor 525, Dibenzylcyclooctyne-Fluor 585, and Dibenzylcyclooctyne-Biotin Conjugate.

17. The method according to any of claims 1 to 9, wherein the Staudinger ligation is an azide-phosphine conjugation.

18. The method according to claim 17, wherein the phosphine is an ester-derivatized phosphine moiety selected from the group consisting of substituted alkyl- and triarylphosphines.

19. The method according to claim 18, wherein the substituted alkyl- and triarylphosphines are selected from the group consisting of triphenylphosphine, 2,2'-bis(diphenylphosphino)-1,1'-binaphthyl and triisopropylphosphine that are modified with various labels, respectively, preferably DyLight 488-Phosphine, DyLight 549-Phosphine, or DyLight 649-Phosphine.

20. A kit for the functionalization of RNA oligonucleotides by enzymatic incorporation of a convertible azide modified nucleotide in a first step, which is in a second step conjugated with an azide-reactive moiety by a copper-catalyzed click reaction, by a copper-free click reaction, or by Staudinger ligation.


SEQUENCE LISTING

<110>  Universität Heidelberg

<120>  Functionalization of RNA oligonucleotides

<130>  U1093EP

<160>  7

<170>  PatentIn version 3.3

<210>  1
<211>  30
<212>  RNA
<213>  artificial sequence

<220>
<223>  oligonucleotide

<400>  1
gugaccgcgg aucgacuuca ccgcgcagug                                        30


<210>  2
<211>  22
<212>  RNA
<213>  artificial sequence

<220>
<223>  oligonucleotide

<400>  2
gcaagcugac ccugaaguuc au                                                22


<210>  3
<211>  22
<212>  RNA
<213>  artificial sequence

<220>
<223>  oligonucleotide

<400>  3
gaacuucagg gucagcuugc cg                                                22


<210>  4
<211>  34
<212>  RNA
<213>  oligonucleotide

<400>  4
gcccgacgau gcuucagcaa ccaguguaau ggcg                                   34


<210>  5
<211>  64
<212>  DNA
<213>  artificial sequence

<220>
<223>  oligonucleotide

<400>  5
cgccattaca ctggttgctg aagcatcgtc gggccactgc gcggtgaagt cgatccgcgg      60

tcac                                                                    64

<210>  6
<211>  54
<212>  DNA
<213>  artificial sequence

<220>
<223>  oligonucleotide

<400>  6
cgccattaca ctggttgctg aagcatcgtc gcggtgaagt cgatccgcgg tcac          54


<210>  7
<211>  17
<212>  DNA
<213>  artificial sequence

<220>
<223>  oligonucleotide

<400>  7
ctgtaggcac catcaat                                                   17